(19) Europäisches Patentamt
European Patent Office
Office européen des brevets

(11) **EP 3 796 834 B1**

(12) **EUROPÄISCHE PATENTSCHRIFT**

(45) Veröffentlichungstag und Bekanntmachung des Hinweises auf die Patenterteilung:
**25.01.2023 Patentblatt 2023/04**

(21) Anmeldenummer: **20726286.6**

(22) Anmeldetag: **12.05.2020**

(51) Internationale Patentklassifikation (IPC):
**A61B 5/022** (2006.01) **A61B 5/0225** (2006.01)
**A61B 5/00** (2006.01)

(52) Gemeinsame Patentklassifikation (CPC):
**A61B 5/02241; A61B 5/02255; A61B 5/7221;**
A61B 2560/0233

(86) Internationale Anmeldenummer:
**PCT/AT2020/060194**

(87) Internationale Veröffentlichungsnummer:
**WO 2020/232492 (26.11.2020 Gazette 2020/48)**

(54) **VERFAHREN UND VORRICHTUNG ZUR VALIDIERUNG EINES BLUTDRUCKMESSSYSTEMS**

METHOD AND DEVICE FOR VALIDATING A BLOOD PRESSURE MEASUREMENT SYSTEM

PROCÉDÉ ET DISPOSITIF DE VALIDATION D'UN SYSTÈME DE MESURE DE LA PRESSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2019 AT 504692019**

(43) Veröffentlichungstag der Anmeldung:
**31.03.2021 Patentblatt 2021/13**

(73) Patentinhaber: **CNSystems Medizintechnik GmbH**
**8020 Graz (AT)**

(72) Erfinder:
• **FORTIN, Jürgen**
**8043 Graz (AT)**

• **FELLNER, Christian**
**8010 Graz (AT)**
• **GROND, Julian**
**8010 Graz (AT)**
• **LERCHE, Katja**
**8081 Pirching am Traubenberg (AT)**
• **BRUNNER, Thomas**
**8071 Grambach (AT)**

(74) Vertreter: **Babeluk, Michael**
**Florianigasse 26/3**
**1080 Wien (AT)**

(56) Entgegenhaltungen:
**WO-A1-2011/051819 US-A1- 2015 201 852**

**Beschreibung**

Die Erfindung betrifft ein Verfahren und eine Vorrichtung zur Validierung eines kontinuierlich messenden, nicht-invasiven Blutdruckmesssystems, das mit einem plethysmographischen System ausgestattet ist, das zumindest eine Lichtquelle und zumindest einen Lichtdetektor aufweist und geeignet ist - in einer Messphase - ein plethysmographisches Signal an einer Extremität zu gewinnen, das einem Regelmechanismus zugeführt wird, welcher einen Anpressdruck an der Extremität verändert, wobei basierend auf dem Anpressdruck der Verlauf des arteriellen Blutdrucks kontinuierlich bestimmt wird.

Die vorliegende Erfindung beschreibt Testsysteme bzw. Simulatoren sowie die dazugehörigen Validierungsverfahren für kontinuierlich messende, nicht-invasive Blutdruckmessgeräte (Continuous Non-invasive Arterial Pressure CNAP). Die Messverfahren der CNAP-Geräte werden oft auch "Penaz Principle", "Vascular Unloading Technique" oder "Volume Clamp Method" genannt, wobei im Folgenden beschriebene neuere Messmethoden über diese Grundprinzipien hinausgehen.

Diese CNAP-Geräte erfassen das Blutdrucksignal eines Lebewesens in Echtzeit, ohne dass ein Katheter in das arterielle Gefäßsystem eingeführt werden muss. Die Genauigkeit dieser CNAP-Geräte im Vergleich zum tatsächlichen intra-arteriellen Blutdruck hat auf Grund neuartiger Messsensoren sowie neuer Methoden (Algorithmen) in der jüngsten Vergangenheit enorm zugenommen und es ist zu erwarten, dass sie in Zukunft aus dem täglichen klinischen Einsatz nicht mehr wegzudenken sind. Für die Akzeptanz im klinischen Einsatz wird es notwendig sein, die Genauigkeit in regelmäßigen Abständen zu überprüfen. Insbesondere bei der regulatorischen Zulassung und in weiterer Folge des Inverkehrbringens neuer CNAP-Geräte oder deren Algorithmen (Methoden, die in Software abgebildet sind) sind standardisierte Testsysteme, Simulatoren und Validierungsverfahren notwendig.

Bei den CNAP-Systemen für die kontinuierliche Blutdruckmessung wird das Blutdrucksignal an einer Extremität (in der Regel am Finger) mit einem speziellen Verfahren und einer speziellen Vorrichtung aufgenommen. Die Vorrichtung weist Signalaufnehmer für die Erfassung eines sogenannten plethysmographischen Signals (Volumssignal) $v(t)$ sowie Mittel für die Veränderung des Anpressdruckes (Counterpressure) $pc(t)$ der Sensorik auf.

Die Messmethoden für diese kontinuierlichen CNAP-Systeme funktionieren im Wesentlichen nach der folgenden Logik: Zuerst wird der Anpressdruck $p_{co}(t)$ bestimmt, bei dem die Pulsationen im Volumssignal $v(t)$ maximal werden. Gemäß dem sogenannten "oszillometrischen Prinzip" bzw. "Prinzip der maximalen Amplitude" entspricht der Anpressdruck $p_{co}(t)$ an dieser Stelle dem mittleren arteriellen Blutdruck mBP. Die Suche nach den maximalen Pulsationen in $v(t)$ und somit nach mBP, wobei der Anpressdruck $pc(t)$ sukzessive verändert wird, nennt man gemäß Stand der Technik "Open-Loop-Phase".

Ist $p_{co}(t)$ gefunden, dann beginnt die sogenannte "Closed-Loop-Phase". In dieser eigentlichen Messphase versucht das CNAP-System mittels einer geschlossenen Regelschleife das oszillometrische Prinzip kontinuierlich aufrecht zu erhalten. Im Konkreten folgt der Anpressdruck $pc(t)$ von CNAP den natürlichen Schwankungen im tatsächlichen arteriellen Blutdruck $p_A(t)$, wobei stets die Amplitude der Pulsationen maximiert wird.

Die bekannten CNAP Verfahren unterscheiden sich nun darin, wie sichergestellt wird, dass sich das System an der Stelle der maximalen Pulsation befindet:

Das Verfahren zur unblutigen Bestimmung des Blutdruckes wurde 1973 von Jan PENAZ vorgestellt (Digest of the 10th International Conference on Medical and Biological Engineering 1973 Dresden). Die "Vascular Unloading Technique" bzw. "Volume Clamp Method" wird daher auch nach dem Erfinder "Penaz Principle" benannt. Dabei wird ein Finger durchleuchtet und durch eine Servoregelung ein Druck auf dem Finger so angebracht, dass der durch die Durchleuchtung registrierte, ursprünglich pulsatile Fluss mit der Hilfe eines elektro-pneumatischen Regelkreises konstant gehalten wird. Das "Prinzip der maximalen Amplitude" kann solange eingehalten werden, solange keine Veränderungen durch die glatte Gefäßmuskulatur (Vasomotorik) auftreten.

Dieser Nachteil wurde gemäß US 4,510,940 A (WESSELIG) eliminiert, wobei die "Closed-Loop-Phase" regelmäßig unterbrochen wird. In der folgenden "Open-Loop-Phase" wird eine neue kurze Suche nach den maximalen Amplituden nach vorgegebenen Kriterien durchgeführt.

Sowohl PENAZ als auch WESSELING verwenden für deren Methode nur einen Regelkreis, der alle Störgrößen ausregeln soll. In der US 8,114,025 B2 (FORTIN) wird eine neue CNAP-Methode beschrieben, bei der ineinandergreifende konzentrische Regelkreise für jeweils eine spezifische Störgröße verantwortlich sind. In der US 8,814,800 B2 (FORTIN) wird das "Prinzip der maximalen Amplitude" weitergehend verbessert; nach jedem Herzschlag wird die Korrektheit des Prinzips überprüft, indem eine spezielle Eigenschaft der Pulsform zur Korrektur des Arbeitspunktes verwendet wird.

In der US 10,098,554 B2 (FORTIN) wird ein neues CNAP-Prinzip beschrieben, das von der einfachen "Volume Clamp Method" abweicht: der Anpressdruck $p_c(t)$ wirkt nun nicht mehr pulsatil auf die Extremität, sondern folgt nur langsam den Änderungen des mittleren arteriellen Blutdrucks mBP. Die pulsatile Natur des kontinuierlichen Blutdruckverlaufes wird aus dem pulsatilen plethysmographischen Signal $v(t)$ gewonnen. In der WO 2016/110781 wird eine Variante eines tragbaren Blutdruckmesssystems (CNAP-2-GO) beschrieben.

Für alle bekannten Systeme gilt, dass sie nur funktionieren, wenn eine Extremität (z.B. der Finger) eines Lebewesens in Kontakt mit der Messsensorik gebracht wird.

Die Aufgabe der Erfindung besteht darin ein Verfahren und eine Vorrichtung zur Validierung eines kontinuierlich messenden, nicht-invasiven Blutdruckmesssystems derart zu verbessern, dass zur Validierung des Blutdruckmesssystems keine Extremität (beispielsweise ein Finger) mit der Vorrichtung in Kontakt gebracht werden muss.

Erfindungsgemäß wird das dadurch erreicht, dass - in einer Validierungs- oder Testphase - ein Signal, abgeleitet von einem zuvor aufgezeichneten Blutdruckverlauf über eine Einkoppelschnittstelle in das Blutdruckmesssystem eingespeist wird, wobei die Lichtstärke der zumindest einen Lichtquelle basierend auf dem zuvor aufgezeichneten Blutdruckverlauf moduliert wird, sowie dass der Einkoppelschnittstelle ein Ausgangssignal aus einem Simulationsmodul zugeführt wird, welchem ein auf einem Speichermedium aufgezeichneter Blutdruckverlauf sowie der Anpressdruck des Blutdruckmesssystems als Eingangssignale zugeführt werden, wobei durch das Blutdruckmesssystem ein simulierter, kontinuierlicher Blutdruckverlauf bestimmt wird.

Vorteilhafte Varianten des erfindungsgemäßen Validierungsverfahrens sind in den abhängigen Verfahrensansprüchen 2 bis 8 dargelegt.

Eine erfindungsgemäße Vorrichtung zur Validierung eines kontinuierlich messenden, nicht-invasiven Blutdruckmesssystems, das mit einem plethysmographischen System ausgestattet ist, das zumindest eine Lichtquelle und zumindest einen Lichtdetektor aufweist und geeignet ist - in einer Messphase - ein plethysmographisches Signal an einer Extremität zu gewinnen, zeichnet sich dadurch aus, dass das Blutdruckmesssystem eine Einkoppelschnittstelle aufweist, über die - in einer Validierungs- oder Testphase - ein Signal, abgeleitet von einem zuvor aufgezeichneten Blutdruckverlauf, in das Blutdruckmesssystem einkoppelbar ist. Die Einkoppelschnittstelle ist geeignet, die Lichtstärke der Lichtquelle basierend auf dem Signal des aufgezeichneten Blutdruckverlaufs zu modulieren. Weiters weist das Blutdruckmesssystem ein Simulationsmodul auf, das eingangsseitig einerseits mit einem Speichermedium verbunden ist, das ein Signal des zuvor aufgezeichneten Blutdruckverlaufs bereit stellt und andererseits mit einer Druckerzeugungseinrichtung des plethysmographischen Systems, die ein Signal des Anpressdrucks bereit stellt, wobei das Simulationsmodul geeignet ist, basierend auf den beiden Eingangssignalen eine Komparatorfunktion zu berechnen und an die Einkoppelschnittstelle zu übermitteln.

Vorteilhafte Varianten der erfindungsgemäßen Vorrichtung sind in den abhängigen Ansprüchen 10 bis 12 dargelegt.

Die erfindungsgemäße Vorrichtung und das erfindungsgemäße Verfahren unterscheiden sich grundsätzlich von bekannten Testsystemen, die für die Validierung von beispielsweise intermittierenden Blutdruckmessgeräten verwendet werden. Diese intermittierenden Blutdruckmessgeräte messen in der Regel am Oberarm oder am Handgelenk den aktuell auftretenden Blutdruck in einer Punktmessung, ohne den kontinuierlichen Verlauf darzustellen. Dabei wird die Manschette über den systolischen Blutdruckwert aufgeblasen und dann der Druck langsam abgelassen. Man erhält dann genau einen systolischen, mittleren oder diastolischen Wert pro Aufblasvorgang, der in der Regel bis zu einer Minute dauert. Die Auswerteverfahren der intermittierenden Blutdruckmessmethoden sind meist das auskultatorische Verfahren nach Riva-Rocci (1896) / Korotkow (1905), bei dem mittels Stethoskop die Geräusche in der Armbeuge abgehört werden, oder das oszillometrische Verfahren, welches häufig in automatisch funktionierenden Geräten eingesetzt wird.

In der Literatur werden Testsysteme für intermittierende Blutdruckmessgeräte beschrieben, in dem die oszillometrischen Impulse bzw. "Korotkow-Töne" künstlich in das zu testende Blutdruckmessgerät eingebracht werden, um so die Messung eines vorgegebenen Blutdruckwertes zu ermöglichen. Diese Testsysteme können für kontinuierliche messende CNAP-Systeme nicht verwendet werden; sie können weder die für diese CNAP-Systeme notwendige Regelschleife schließen noch die physiologischen Eigenschaften der Extremität (z.B. Finger) nachbilden.

Die im eingangs zum Stand der Technik beschriebenen Verfahren haben eine Gemeinsamkeit: das geschlossene CNAP-Regelungssystem versucht in der "Closed Loop Phase" die Volumensveränderungen $v(t)$ bzw. gemäß US 10,098,554 B2 bestimmte Frequenzinhalte von $v(t)$ (bezeichnet als $v_F(t)$) durch Veränderung des Anpressdruckes $pc(t)$ konstant zu halten. Wie später im Detail beschrieben wird, gibt $v(t)$ bzw. geben bestimmte Frequenzinhalte $v_F(t)$ an, wie groß die Abweichung vom aktuellen Anpressdruck $p_c(t)$ zum tatsächlichen arteriellen Blutdruck $p_A(t)$ ist, da sich durch die Abweichungen $pc(t) - p_A(t)$ auch Volumsänderungen ergeben. In der Sprache der System- und Regelungstechnik entspricht $v(t)$ bzw. $v_F(t)$ der Regelabweichung auch als Fehlersignal $e(t)$ bezeichnet. Ist die Regelabweichung $e(t)$ hinreichend klein, so kann $p_A(t)$ hinreichend genau aus $pc(t)$ rekonstruiert werden - natürlich ohne dass man $p_A(t)$ mittels invasiven Katheters bestimmen muss, was der große Vorteil dieser CNAP-Systeme ist. Bei den neueren Verfahren gemäß US 10,098,554 B2 und WO 2016/110781 A1 werden für die Bestimmung des pulsatilen $p_A(t)$ neben $pc(t)$ auch weitere Frequenzinhalte von $v(t)$ benötigt.

Ein wesentliches Merkmal aller CNAP-Systeme ist in weiterer Folge der Umstand, dass sich das vergleichende Element der Regelstrecke - d.h. der Ort wo die "Führungsgröße" $p_A(t)$ mit der "Regelgröße" $pc(t)$ verglichen wird - im Körper bzw. in der Extremität des Lebewesens befindet. Regelungstechnisch gesehen ist es unerheblich, wo sich der sogenannte "Komparator" befindet. Praktisch bedeutet dies jedoch, dass ein herkömmliches CNAP-System nur dann funktioniert, wenn eine Extremität bzw. ein Finger eines Lebewesens zur Verfügung steht - also auch dann, wenn das CNAP-System getestet oder validiert werden soll.

Dem CNAP-System steht somit die genaue Führungsgröße - also der tatsächliche arterielle Blutdruck $p_A(t)$ - nicht zur Verfügung, es erhält nur die plethysmographische Information, d.h. ein Signal, das den Veränderungen des Volumens $v(t)$ der Arterie entspricht. Diese Information wird in der Regel durch ein Lichtverfahren gewonnen, indem auf der einen Seite der Extremität (z.B. Finger) eine Lichtquelle (z.B. LED) angebracht wird und auf der anderen Seite das transmittierte Signal (z.B. mittels Fotodiode) gemessen wird. Die Lichtquelle strahlt dabei bei allen bekannten Systemen ein Licht mit konstanter Lichtstärke aus, bzw. werden in den meisten Fällen konstante, rechteckförmige Lichtimpulse verwendet. Das Verwenden von Lichtimpulsen erlaubt einerseits eine höhere Energiedichte bei gleichzeitig reduziertem Energieaufwand und erlaubt andererseits die Erfassung des Umgebungslichtes während der sog. Austastlücke. Lichtsysteme für CNAP-Geräte werden beispielsweise in der WO 2011/051819 A1 bzw. der US 8,343,062 B2 (FORTIN) beschrieben.

Bisher konnte die Führungsgröße $p_A(t)$ nur dann in ein CNAP-System eingebracht werden, wenn eine Extremität (z.B. Finger) eines Lebewesens vorhanden ist. Logischerweise kann dabei nur der aktuelle Blutdruck der Versuchsperson gemessen werden. Aus regulatorischen Gründen ist dies ein großer Nachteil, denn so können keine standardisierten, wiederholbaren Testmessungen durchgeführt werden. Systematische Untersuchungen des CNAP-Systems sind so nicht möglich, im Gegensatz zu anderen biomedizinischen Systemen wie z.B. dem EKG oder den vorhin angeführten Testsystemen für intermittierende Blutdruckmessgeräte können keine einmal aufgenommenen Signale eingespielt und wiederholt getestet werden. Problematische Blutdruckverläufe wie z.B. Blutdruckeinbrüche während Operationen, Asystolien bei Kipptischuntersuchungen, Veränderungen während unterschiedlicher Herzschrittmachereinstellungen, etc. können nicht reproduziert werden. Veränderungen am CNAP-System müssen aus regulatorischen Gründen validiert werden, was gemäß Stand der Technik nur durch erneute Testmessungen am Patienten in der Klinik möglich ist. Da solche problematischen Blutdruckverläufe nicht vorhersagbar auftreten, sind auch Messungen an einer Vielzahl von Patienten notwendig, um statistisch signifikante Aussagen zu erhalten.

Die vorliegende Erfindung ermöglicht es nun standardisierte Validierungsmessungen durchzuführen. Im Folgenden werden mehrere Ausführungsvarianten von Vorrichtungen und Verfahren zur Validierung von CNAP-Systemen beschrieben.

Eine grundsätzliche Methode zur Einspeisung des zuvor aufgezeichneten Blutdruckverlaufs als Führungsgröße $p_A(t)$ geschieht dabei über eine Modulation des Lichtes, konkret über eine Modulation der LED-Lichtimpulse. Das CNAP-System kann bei der erfindungsgemäßen Vorrichtung nicht mehr unterscheiden, ob das resultierende Lichtsignal $v(t)$ an der Empfangsseite von einem Simulationsmodul der Vorrichtung generiert wurde, oder ob die Modulation von einem Finger einer Person stammt. Das CNAP-System erzeugt in weiterer Folge über die beschriebene Regelstrecke einen Anpressdruck $p_c(t)$ aus dem man den zu bestimmenden arteriellen Blutdruck $p_A(t)$ ableiten kann. Die Regelgröße $p_c(t)$ wird wiederum in das Simulationsmodul eingespielt und mit der weiterlaufenden aufgezeichneten Führungsgröße $p_A(t)$ verglichen. Es wird ein neues $v(t)$ erzeugt und wiederum über eine Modulation des Lichtes in das System eingespielt und der Regelkreis wurde geschlossen.

So ist es möglich, dass ein klinisch aufgezeichneter Blutdruckverlauf - genauer gesagt das einmal aufgenommene Blutdrucksignal eines Patienten - in das zu testende CNAP-System eingespielt werden kann. Das daraus entstehende Messsignal kann nun mit dem Eingangssignal verglichen werden. Es ist dabei unerheblich, ob das Patientensignal von einem intra-arteriellen Katheter stammt, oder ob es von einem CNAP-Gerät stammt, das während der klinischen Messung verwendet wurde.

Die Erfindung beschreibt weiters Validierungsmethoden von CNAP-Systemen, die mit dem vorliegenden Testsystem bzw. Simulator durchgeführt werden können sowie Methoden, die eine Äquivalenz von bereits zugelassenen CNAP-Systemen mit neuen, modifizierten Systemen nachweisen können.

Die Erfindung wird im Folgenden an Hand von Ausführungsbeispielen näher erläutert. Es zeigen:

Fig. 1        ein Blutdruckmesssystem in schematischer Darstellung mit einen CNAP-Regelkreis gemäß dem Stand der Technik in der Messphase;

Fig. 2        eine erfindungsgemäß Validierungsvorrichtung mit einem Blutdruckmesssystem gemäß Fig. 1;

Fig. 3a        und Fig. 3b Diagramme von LED-Pulsen;

Fig. 4a        und 4b p-v-Diagramme zur Ermittlung des Setpoints; sowie

Fig. 5a        bis 5d Validierungsverfahren mittels "Toleranzdreieck".

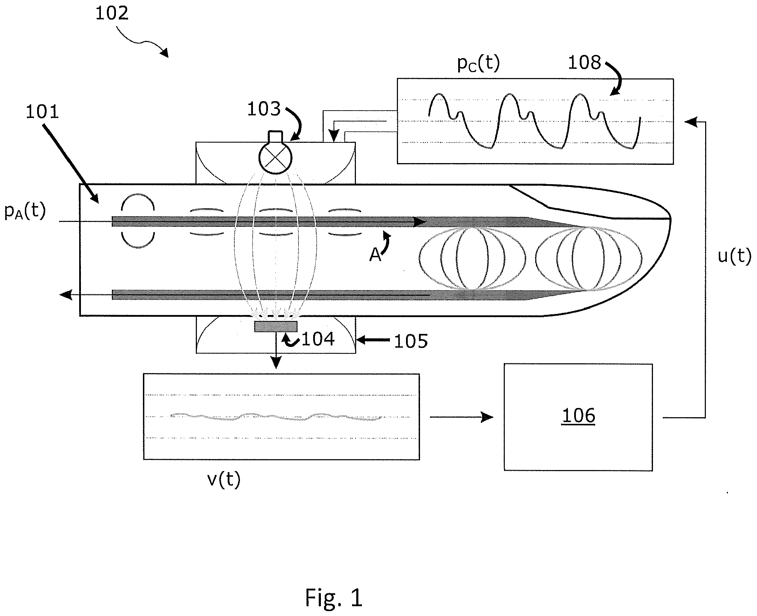

Fig. 1 zeigt ein Blutdruckmesssystem 102 gemäß Stand der Technik welches für kontinuierliche nicht-invasive CNAP-Methoden zur Bestimmung des Blutdrucksignales $p_A(t)$ folgende regeltechnische Verfahren angewendet: An eine Extremität 101 oder eine Körperstelle eines Lebewesens, in der sich eine Arterie A befindet - wie zum Beispiel der Finger 101, eine Handwurzel oder Schläfe - wird ein plethysmographisches System 103, 104, beispielsweise in Form einer Fingermanschette 105 angebracht und so mittels einer Lichtquelle 103 durchleuchtet. Das Licht, das diese Extremität 101 durchströmt oder am in der Extremität liegenden Knochen reflektiert wird (z.B. Handwurzel, Schläfe), wird mit einem geeigneten Lichtdetektor 104 registriert und ist ein inverses Maß für das arterielle Blutvolumen in der Extremität (plethysmographisches Signal $v(t)$). Je mehr Blut sich in der Extremität befindet, desto mehr Licht wird absorbiert und desto kleiner ist das plethysmographische Signal $v(t)$.

Das Signal $v(t)$ wird nun einem Regelmechanismus 106 zugeführt und ein Stellwert $u(t)$ wird bestimmt, der in weiterer Folge den von einer Druckerzeugungseinheit 108 erzeugten Anpressdruck $pc(t)$ an der Extremität 101 verändert. Der Anpressdruck $pC(t)$ wirkt an jener Stelle, wo auch das plethysmographische Signal $v(t)$ bestimmt wird. Die Bedingung des Regelmechanismus legt fest, dass $v(t)$ bzw. bestimmte Frequenzinhalte von $v(t)$ (bezeichnet als $v_F(t)$) durch den anliegenden Anpressdruck $pc(t)$ konstant gehalten werden. Wenn sich somit $v(t)$ verändern will, dann stellt der Reglermechanismus 106 über den Stellwert $u(t)$ den Druck $p_c(t)$ derartig nach, dass $v(t)$ weiterhin konstant bleibt.

Wird diese Regelbedingung eingehalten - also $v(t)$ und somit das Blutvolumen in der Extremität 101 bleiben über die Zeit konstant - dann ist der Druckunterschied zwischen dem intraarteriellen Druck $p_A(t)$ und dem außen anliegenden Anpressdruck $pc(t)$ - der sogenannte transmurale Druck $p_T(t)$ - gleich Null. Somit entspricht der Anpressdruck $p_c(t)$ dem intraarteriellen Druck $p_A(t)$ in der Extremität. Dieser von der Druckerzeugungseinheit 108 bereit gestellte Anpressdruck $p_c(t)$ kann mittels eines Drucksensors bzw. Manometers gemessen werden.

Eine wesentliche Komponente, nämlich der Komparator der Regelstrecke befindet sich im Inneren der Extremität (z.B. Finger). Der sogenannte Komparator ist das Vergleichselement, in dem die "Führungsgröße" $p_A(t)$ mit der "Regelgröße" $pc(t)$ verglichen wird. Wenn sich $p_A(t)$ gegenüber $pc(t)$ verändert, dann entstehen Volumenschwankungen, die über das plethysmographische Signal $v(t)$ erfasst werden.

Die Regelschleife gemäß Fig. 1 funktioniert somit nur, wenn auch der menschliche Komparator - also die Extremität 101 bzw. der Finger - in das CNAP-System eingeführt wird. Dieser menschliche Komparator in Form einer Testperson, genauer gesagt die Komparatorfunktion - musste bisher auch vorhanden sein, wenn die Geräte überprüft bzw. validiert wurden. Der aktuelle Blutdruck in der Arterie der Testperson $p_A(t)$ wird somit mit dem Anpressdruck $pc(t)$ verglichen und die grundsätzliche Komparatorfunktion

$$v(t) = \mathrm{f}\left(p_C(t) - p_A(t)\right) \qquad (1)$$

wird erzeugt. Der aktuelle Blutdruck in der Arterie $p_A(t)$ liegt auch nicht dem CNAP-System direkt vor, was ja der Vorteil dieses nicht-invasiven CNAP-Systems ist. Dadurch ist es aber auch - im Vergleich zu anderen Testsystemen wie z.B. für EKG oder den vorhin angeführten Testsystemen für intermittierende Blutdruckmessgeräte - nicht möglich, dass einfach ein biologisches Signal an einem Messsensor eingebracht wird und so das zu testende Gerät einen Messwert erzeugt. Das einem CNAP-System zur Verfügung stehende biologische Signal wird ja unmittelbar durch die Messmethode bzw. dem Anpressdruck $p_c(t)$ beeinflusst und verändert.

Für die Überprüfung, Testung und Validierung von CNAP-Systemen ist es vorteilhaft, wenn die Komparatorfunktion standardisiert zur Verfügung gestellt werden kann, was im Folgenden anhand von Ausführungsvarianten von Validierungsverfahren und Validierungsvorrichtungen beschrieben wird.

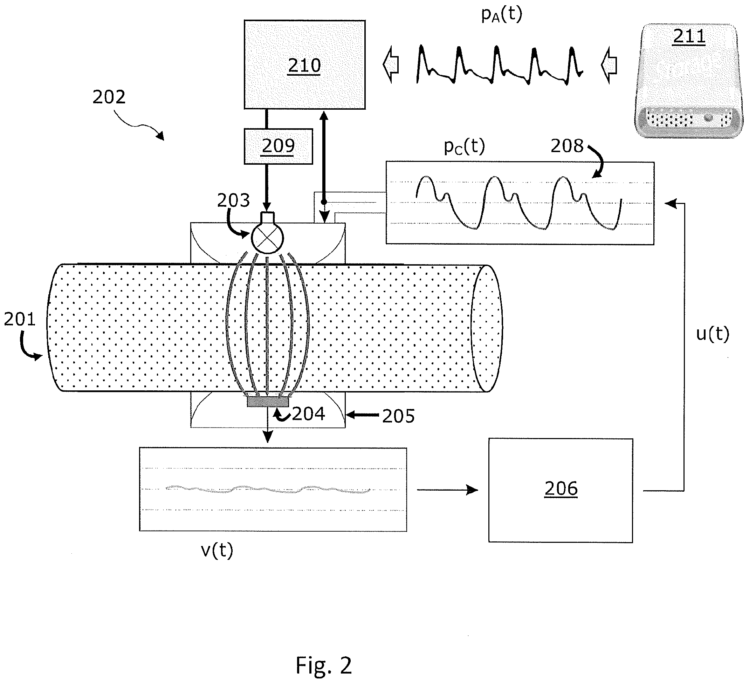

Figur 2 zeigt ein Blockschaltbild der erfindungsgemäßen Validierungsvorrichtung. Anstelle der Extremität bzw. des Fingers befindet sich ein einfacher Fingerdummy 201 in Kontakt mit dem plethysmographischen System. Das plethysmographische Signal $v(t)$ wird mittels der Lichtquelle 203 und einem Lichtsensor 204 erzeugt. Das Signal $v(t)$ wird dem Regelmechanismus 206 zugeführt und ein Stellwert $u(t)$ bestimmt, der in weiterer Folge einen Anpressdruck $p_c(t)$ verändert.

Bei dieser Ausführungsvariante wird darauf geachtet, dass dieselben CNAP-Komponenten aktiv sind, wie sie auch gemäß Stand der Technik aus Figur 1 verwendet werden. Dies ist für den später beschriebenen Validierungsnachweis notwendig. Idealerweise sind die CNAP-Geräte so ausgeführt, dass sie in einen Simulatormodus versetzt werden und sich so quasi selbst validieren können. Es entsprechen somit die Komponenten des Blutdruckmesssystems 202 in Figur 2 jenen des Blutdruckmesssystems 102 aus der Figur 1; die Lichtquelle 203 der Lichtquelle 103, usw. Die ergänzenden Systemkomponenten sind gemäß Figur 2 folgendermaßen erklärt:

Der Fingerdummy 201 ist primär dafür verantwortlich, dass der von der Druckerzeugungseinheit 208 bereit gestellte Anpressdruck $p_c(t)$ im Blutdruckmesssystem 202 auch tatsächlich auftreten kann, indem der Anpressdruck $pc(t)$ auf ein Gegenstück trifft. Oft wird der Anpressdruck $pc(t)$ mittels aufblasbarer Fingermanschette 205 erzeugt, diese Manschette könnte sogar platzen, wenn kein Gegenstück vorhanden ist.

Weiters soll der Fingerdummy 201 ähnliche haptische Eigenschaften aufweisen, wie ein tatsächlicher Finger. Insbesondere die Viskositätseigenschaften sollen nachgebildet werden, indem z.B. ein Gel oder Gelatine als Material für den Fingerdummy 201 verwendet wird, das mit einer elastischen Haut umgeben ist. Das ist vorteilhaft um die Ankoppelung der Lichtquelle 203 und Lichtsensoren 204 zu simulieren.

Der Fingerdummy 201 weist bevorzugt ähnliche optische Eigenschaften auf, wie ein tatsächlicher Finger eines Lebewesens. Die Absorptionskonstante des Fingerdummies 201 wird beispielsweise für die in der Lichtquelle 203 auftretende Lichtwellenlänge so gewählt, wie sie in einem Finger auftritt, der mit einem Anpressdruck pc(t) über den systolischen Blutdruck abgepresst ist. Die Lichtmenge, die durch einen Finger durchgeleuchtet wird, welcher über den systolischen Blutdruck abgeschnürt wird, sollte mindestens auch durch den Fingerdummy 201 übertragen werden können, aber nicht wesentlich mehr.

Der Fingerdummy 201 erfüllt somit haptisch und optisch Mindestanforderungen; er muss jedoch im Gegensatz zu einem tatsächlichen Finger nicht das Lichtsignal modulieren, um so die Information über den arteriellen Blutdruckverlauf $p_A(t)$ in das CNAP-System einzubringen.

Das soll erfindungsgemäß über eine Modulation der Lichtquelle 203 funktionieren. In der Messphase gemäß Figur 1 leuchtet die Lichtquelle 103 stets mit konstanter Lichtenergie. Diese Lichtenergie soll nun in einer Validierungs- oder Testphase über eine Einkoppelschnittstelle 209 verändert bzw. moduliert werden können. Das so modulierte Licht strömt nun durch den Fingerdummy 201 und trifft auf den Lichtsensor 204. Dieser empfängt das Licht unabhängig davon, ob die Lichtmodulationen durch Veränderungen der Absorption in einem Finger entstehen, oder durch Modulationen der Lichtstärke in der Lichtquelle 203. In weiterer Folge wird das resultierende Signal v(t) dem Regelmechanismus 206 zugeführt, der einen Stellwert u(t) bestimmt, sowie in der weiteren Folge der Anpressdruck $p_c(t)$ verändert, welcher wiederum auf den Fingerdummy 201 wirkt.

Nun fehlt noch das für jedes Regelsystem notwendige Vergleichselement bzw. ein Komparator, in dem die Führungsgröße $p_A(t)$ mit der Regelgröße $p_c(t)$ verglichen wird. Dafür ist ein Simulationsmodul 210 vorgesehen, das als Eingangsparameter einerseits den Anpressdruck pc(t) des Blutdruckmesssystems 202 erhält, sowie einen auf einem Speichermedium 211 zuvor aufgezeichneten Blutdruckverlauf $p_A(t)$. Das Simulationsmodul 210 berechnet das plethysmographische Signal v(t) gemäß Gleichung (1) - der sogenannten Komparatorfunktion des Simulationsmoduls 210 - und führt es der Einkoppelschnittstelle 209 zu, um die Modulation des Lichtes in der Lichtquelle 203 zu gewährleisten.

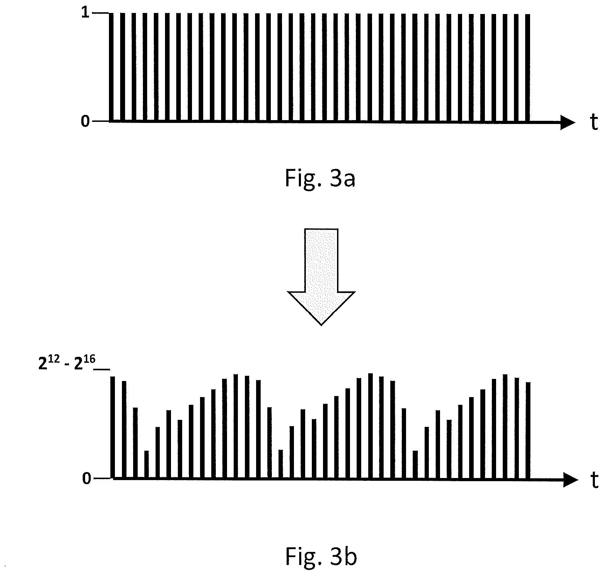

Die Figuren 3a und 3b zeigen die Veränderungen in der Ansteuerung der Lichtquelle 103 (Fig.1: Messphase bzw. Stand der Technik) bzw. 203 (Fig. 2: Validierungsphase der Erfindung) auf, wobei bevorzugt LEDs als Lichtquellen 103, 203 verwendet werden. Es ist bekannt, dass die Signalausbeute einer LED besser wird, wenn man die LED mit elektronischen Pulsen ansteuert. Man kann so kurzfristig höhere LED-Ströme erzeugen, obwohl in Summe der Energieverbrauch der LED reduziert wird. Die Information über die Einschaltzeitpunkte der Pulse werden dem Lichtempfänger 104, 204 mitgeteilt. Figur 3a zeigt solche elektronischen Ansteuerpulse während der Messphase. Es gibt nur Ansteuerpulse, die entweder die Lichtquelle 103 zur Gänze einschalten (Zustand 1) oder ausschalten (Zustand 0).

Figur 3b zeigt nun, wie die Energie der Einschaltimpulse moduliert werden kann. Damit die vollständige Information eines Blutdruckverlaufes moduliert und somit über die Lichtquelle 203 in das CNAP-System übertragen werden kann, sollte die Höhe des elektronischen Pulses mit einer zumindest 12 Bit-Kodierung bis maximal ca. 16 Bit-Kodierung ausgestattet sein. Alle anderen Kodierungen sind erfindungsgemäß ebenso möglich.

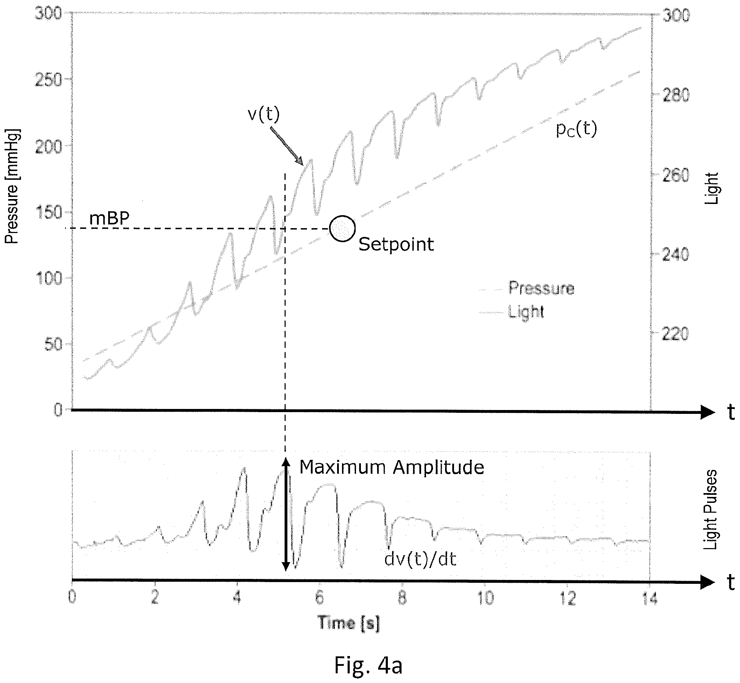

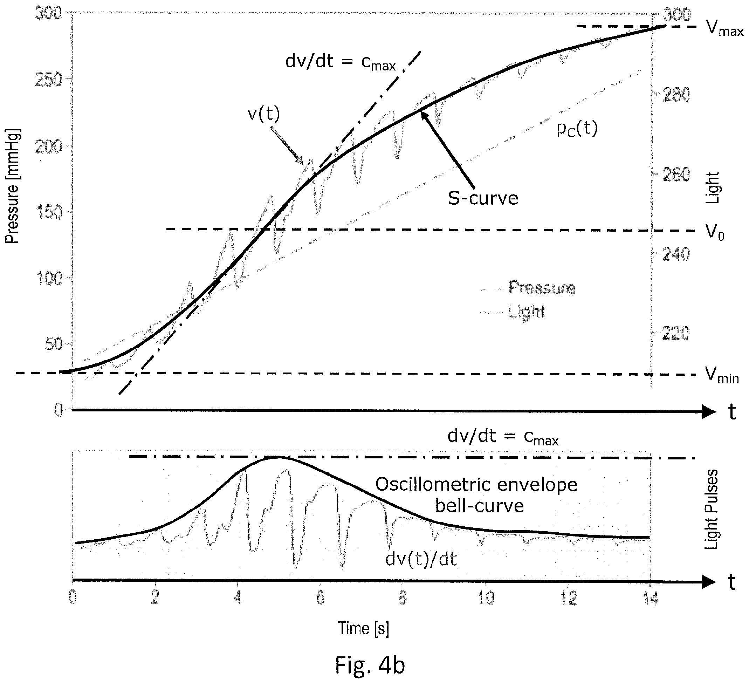

Figuren 4a sowie 4b zeigen ein typisches Druck-Volumendiagramm (p-v-Diagramm). Diese Information ist notwendig um die Funktion aus Gleichung (1) zu ermitteln und so die Komparatorfunktion des Simulationsmoduls 210 in weiterer Folge zu beschreiben. Das p-v-Diagramm wird in der Regel in der Open-Loop-Phase bestimmt, also am Beginn jeder Messung, wo gemäß dem "oszillometrischen Prinzip" bzw. dem "Prinzip der maximalen Amplitude" nach den maximalen Pulsationen und somit nach dem mittleren arteriellen Blutdruck mBP gesucht wird.

In Figur 4a wird gezeigt, dass an der Stelle:

$$v(t) = \text{maximale Pulsationen} / \text{Anpressdruck } p_{Co}(t) = mBP$$

der Arbeitspunkt (Setpoint) gefunden wurde. Dazu wurde der Anpressdruck $p_c(t)$ - in diesem Beispiel anhand einer Rampenfunktion - verändert und das dazugehörige plethysmographische Signal v(t) ermittelt. Man sieht, dass v(t) eine pulsatile Komponente aufweist sowie eine Art von "Gleichanteil". Die pulsatile Komponente entsteht durch die arteriellen Blutdruckschwankungen und deren Frequenz entspricht naturgemäß der Herzfrequenz. Der sogenannte Gleichanteil hingegen kommt durch die Absorption des Lichtes durch Knochen, Haut, Gewebe sowie dem venösen Blut, der interstitiellen Flüssigkeit und auch durch den Füllungsgrad der Arterie(n). Das venöse Blut und die interstitielle Flüssigkeit lassen sich durch den Anpressdruck pc(t) verdrängen, deswegen verändert sich der Gleichanteil dieser Anteile auch mit dem Anpressdruck pc(t). Besonders wesentlich verändert sich der Gleichanteil auch durch das Komprimieren der Arterie(n), die sich in der Extremität wie z.B. dem Finger befinden.

Um die pulsatile Komponente aus v(t) zu ermitteln, wird das Signal mit einem Hochpass gefiltert. Mathematisch gesehen entspricht dieser Vorgang einfach gesagt dem Ermitteln der ersten Ableitung nach der Zeit. Mit steigendem Druck $p_c(t)$ wachsen zunächst die Amplituden der Pulse an, um ab einem bestimmten Druck - wie vorhin beschrieben ab dem mittleren arteriellen Blutdruck mBP bzw. an der Stelle $p_{Co}(t)$ - wieder kleiner zu werden. Die Amplituden der Pulse verhalten sich gemäß einer Glockenkurve. Es entsteht ein typisches Bild der "oszillometrischen Einhüllenden", wie sie auch von automatischen intermittierenden Blutdruckmessgeräten bekannt ist.

Der Gleichanteil ist bei niedrigem Anpressdruck $p_c(t)$ am kleinsten, denn das Licht wird neben Knochen, Haut und Gewebe, venösen Blut und der interstitiellen Flüssigkeit auch wesentlich vom arteriellen Blut absorbiert, das sich - über die Pulsationen gemittelt - in der Arterie befindet. Mit steigendem Druck steigt auch der Gleichanteil und bei einem Anpressdruck $p_c(t)$ weit über dem systolischen Blutdruck sBP ist das arterielle Blut vollständig aus der Arterie verdrängt und es kommt zu einer Sättigung. Das Licht wird nur mehr von Knochen, Haut und Gewebe absorbiert. Der Gleichanteil erreicht ein Maximum.

In Figur 4b wird dasselbe p-v-Diagramm noch detaillierter dargestellt. Der Gleichanteil von v(t) wurde bestimmt und man sieht, dass dieser einer S-Kurve entspricht. Diesen S-kurvenförmigen Gleichanteil kann man auch durch die mathematische Integration der glockenkurvenförmigen "oszillometrischen Einhüllenden" ermitteln. Die maximale Steigung der S-Kurve $c_{max}$ entspricht der maximalen Amplitude bzw. der Höhe der Glockenkurve.

Die gesuchte Komparatorfunktion für den Finger entspricht im Wesentlichen dem S-kurvenförmigen Verlauf des Gleichanteils. Die erste Ableitung dieser Komparatorfunktion entspricht auch der oszillometrischen Glockenkurve aus der Verteilung der Pulsamplituden. Wie in Figur 4b gezeigt, können für die Parametrisierung der S-Kurve in einer vorliegenden Ausführungsvariante der Komparatorfunktion die Extremwerte $V_{min}$ (Gleichanteil, wenn $p_C(t) = 0$) und $V_{max}$ (Gleichanteil, wenn $pc(t) \gg sBP$) sowie der Arbeitspunkt bei $V_0 / p_{Co}(t) = mBP$ herangezogen werden. Die Steigung an der Stelle des Arbeitspunktes entspricht $c_{max}$ gemäß Figur 4b.

Es lässt sich folgendes Gleichungssystem für die Komparatorfunktion aufstellen:

$$v(t) = \begin{cases} V_{min} + (V_0 - V_{min}) \cdot e^{\frac{c_{max}}{V_0 - V_{min}} \cdot (p_C(t) - p_A(t))} & \forall\, p_C(t) < p_A(t) \\ V_{max} - (V_{max} - V_0) \cdot e^{-\frac{c_{max}}{V_0 - V_{min}} \cdot (p_C(t) - p_A(t))} & \forall\, p_C(t) > p_A(t) \end{cases} \qquad (2)$$

Die Steigung k verhält sich in diesem Fall gemäß:

$$k(t) = \frac{dv}{dp} = \begin{cases} c_{max} \cdot e^{\frac{c_{max}}{V_0 - V_{min}} \cdot (p_C(t) - p_A(t))} & \forall\, p_C(t) < p_A(t) \\ c_{max} \cdot e^{-\frac{c_{max}}{V_0 - V_{min}} \cdot (p_C(t) - p_A(t))} & \forall\, p_C(t) > p_A(t) \end{cases} \qquad (3)$$

Erfindungsgemäß können auch andere S-kurvenförmige Funktionen verwendet werden, wie z.B. der Areasinus hyperbolicus (arsinh) oder die Gaußsche Verteilungs- oder Summenfunktion Φ, die in geeigneten Varianten dieser Funktionen angewendet werden können.

In weiteren Ausführungsvarianten kann die Komparatorfunktion aus anderen mathematischen Methoden bestimmt werden. So bietet sich z.B. die Vermessung der v(t)-Amplituden bei unterschiedlichen Anpressdrücken $p_c(t)$ an, wobei dann diese v(t)-Amplituden als Stützstellen für jede Art von empirischen Funktionen dienen können. Die unterschiedlichen v(t)-Amplituden bei verschiedenen Anpressdrücken pc(t) können in einer Open-Loop-Phase am Beginn der Messung ermittelt werden.

Experimente haben gezeigt, dass es auch vorteilhaft ist weitere physiologische Eigenschaften der Komparatorfunktion abzubilden. Wie vorhin beschrieben ist der Gleichanteil von v(t) auch vom venösen Blut sowie der interstitiellen Flüssigkeit abhängig, denn beide Anteile sind ebenso vom Anpressdruck $p_c(t)$ abhängig. Die Anteile des venösen Blutes verschwinden relativ rasch, wenn der Anpressdruck über den venösen Blutdruck - also auf $p_C(t) \gg 20$ mmHg - ansteigt, wo ja auch der Betriebsdruck von CNAP-Geräten in der Regel liegt.

Beim Anteil der interstitiellen Flüssigkeit ist der Fall anders. Erstens erzeugen pulsatile Veränderungen von pc(t) ein ständiges Rausquetschen sowie Reinsaugen von interstitieller Flüssigkeit in den Messfinger. Das wirkt sich so auf v(t) aus, dass sich eine Hysterese bildet.

Diese Hysterese kann folgendermaßen mathematisch abgebildet werden:

$$v_{hys}(t) = \mathrm{v}(t) - c_{hys} \cdot k(t) \cdot \frac{d(p_C(t) - p_A(t))}{dt} \qquad (4)$$

wobei $v_{hys}(t)$ das mit Hysterese behaftete plethysmographische Signal ist und $c_{hys}$ eine für die Hysterese charakteristische Konstante. v(t) ist das plethysmographische Signal, das aus einer der vorhin beschriebenen Methoden bestimmt wurde; k(t) ist die erste Ableitung dv(t)/dt.

Zweitens dauert es erfahrungsgemäß bis zu fünf Minuten bis die interstitielle Flüssigkeit soweit aus dem Finger gepresst wurde, bis sich ein stabiles Gleichgewicht einstellt. Solange der Gleichanteil wegen des interstitiellen Flüssigkeitsanteiles driftet, kommt es zu einer Art Parallelverschiebung der S-kurvenförmigen Komparatorfunktion. Solche "Fluid Shifts" können auch immer wieder während des Betriebs auftreten, z.B. durch Umlagerung des Messfingers oder nach Aufnahme von Flüssigkeit durch den Patienten durch Trinken, aber auch durch Infusionen. Deswegen ist es vorteilhaft, die S-Kurve bzw. deren Parameter in regelmäßigen Abständen neu zu vermessen. Auch Vasomotorik beeinflusst die S-kurvenförmigen Komparatorfunktion, was eine Überprüfung und ggf. eine Korrektur sinnvoll macht.

Für die Überprüfung und ggf. einer Korrektur der Komparatorfunktion während des Betriebes ist die Bestimmung von v(t)-Amplituden bei unterschiedlichen Anpressdrücken pc(t) in einer kurzen Open-Loop-Phase, aber auch Evaluationssequenzen während der Closed-Loop-Phase vorstellbar.

Liegt die S-kurvenförmigen Komparatorfunktion vor und wird diese gemäß einer Ausführungsvariante auch noch regelmäßig überprüft und ggf. korrigiert, kann man die Regelschleife des vorliegenden Simulators wie in Figur 2 beschrieben schließen. Dieser Regelkreis sieht zusammengefasst wie folgt aus: auf einem Speichermedium 211 ist ein arterieller Blutdruckverlauf $p_A(t)$ gespeichert. Dieser wird ausgelesen und im richtigen zeitlichen Verlauf dem Simulationsmodul 210 zugeführt, in dem die S-kurvenförmige Komparatorfunktion abgebildet ist. Weiters ist der Anpressdruck pc(t) eine Eingangsvariable des Simulationsmoduls 210. Das Ausgangssignal des Simulationsmoduls 210 ist das plethysmographische Signal v(t), mit dem nun die Lichtquelle 203 moduliert wird. Das Licht strömt durch den Fingerdummy 201 und trifft auf den Lichtsensor 204. Dieser Lichtsensor 204 kann nicht unterscheiden, ob das bei ihm ankommende Signal v(t) durch Absorption in einem Finger moduliert wurde, oder durch das Simulationsmodul. Das Signal v(t) wird dem Regelmechanismus 206 zugeführt, der Stellwert u(t) bestimmt, sowie in der weiteren Folge der Anpressdruck pc(t) - verändert, welcher wiederum auf den Fingerdummy 201 wirkt. Das Signal pc(t) wird ebenso dem Simulationsmodul 210 zugeführt, wo dieser wieder mit dem auf dem Speichermedium 211 befindlichen arteriellen Blutdruckverlauf $p_A(t)$ verglichen wird.

Nota bene: das Simulationsmodul 210 dieser Ausführungsvarianten erzeugt analoge Signale: sowohl das Licht wird moduliert und dadurch ein Signal v(t) erzeugt, weiters ist auch der Anpressdruck pc(t) im Blutdruckmesssystem 202, beispielsweise in der Fingermanschette 205 wirksam.

In anderen Ausführungsvarianten kann der Regelkreis auch digital auf einem Computer nachgebildet werden. Dabei müssen gewisse elektronische Elemente wie z.B. das plethysmographische System oder die Mittel zur Veränderung des Anpressdruckes pc(t) digital nachgebildet werden. Dies hat den Vorteil, dass andere wichtige digitale Elemente wie z.B. der Regelmechanismus 206, der hauptsächlich in Form eines Algorithmus in Software-Codes vorliegt, einfach auf einem Computer getestet werden können.

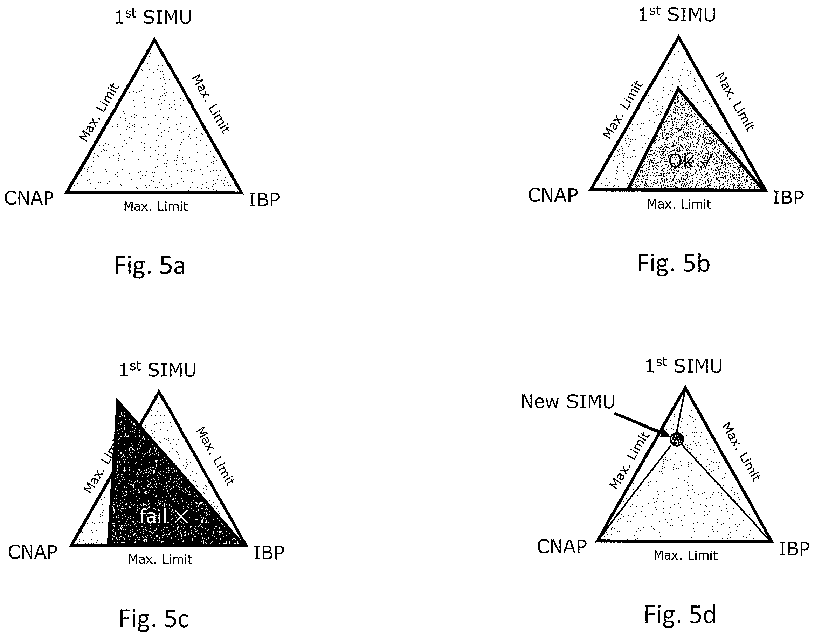

Die Figuren 5a bis 5d beschreiben mögliche Methoden zur Validierung. Dabei muss zunächst erwähnt werden, dass die hier beschriebenen Testsysteme und Simulatoren nur dann effizient für Validierungszwecke verwendet werden können, wenn auch die Funktionalität der Testsysteme und Simulatoren validiert werden kann. Es muss nachgewiesen werden, dass die Systeme hinreichend genau funktionieren.

Für medizintechnische Geräte im Allgemeinen und für Blutdruckmessgeräte im Speziellen gelten Normen, die Toleranzgrenzen festgelegt haben. Diese Toleranzgrenzen müssen neue Geräte im Vergleich zu Gold-Standard-Methoden einhalten, wenn sie auf den Markt gebracht werden. So gilt z.B. für intermittierende Blutdruckmessgeräte eine mittlere Differenz zum Gold-Standard von 5 mmHg und eine Standardabweichung von 8 mmHg. Für kontinuierliche Blutdruckmessgeräte wird derzeit an einer eigenen Norm gearbeitet.

Um den Nachweis zu liefern, dass das vorliegendes Testsystem ebenfalls diese Norm und die Toleranzgrenze einhält, könnte folgendermaßen vorgegangen werden: Bei der Aufzeichnung eines Blutdruckverlaufes, der später auch in ein CNAP-Simulatorsystem eingespielt werden kann, sollen simultan der wahre intra-arterielle Blutdruck (IBP) mittels Katheter (Gold-Standard) sowie ein nicht-invasiver Blutdruckverlauf (CNAP) aufgenommen werden. Idealerweise sollten Blutdruckverläufe nicht nur bei einem Patienten aufgenommen werden, sondern bei einer gewissen Anzahl von Patienten, wie sie auch in der Norm festgelegt werden. Aus der statistischen Auswertung entsteht eine Abweichung zwischen CNAP und dem Gold-Standard IBP, die idealerweise innerhalb der vorgeschriebenen Toleranzgrenze zu liegen kommt.

Genau dieses für die Aufzeichnung verwendete CNAP-System inklusive aller dazugehöriger Komponenten wird nun zum Simulator umgebaut. Die Komparatorfunktion des jeweiligen Patienten wird in das Simulationsmodul 210 eingespielt und mit dem dazugehörigen IBP wird die erste Simulatoraufzeichnung erzeugt - in Figuren 5a-d als "1st SIMU" bezeichnet. Dies wird für alle Patientenfiles durchgeführt und es entsteht ein Datenset von 1st SIMUs. Dieses Datenset kann nun sowohl mit dem IBP-Datenset als auch mit dem CNAP Datenset verglichen werden. Die Summe der Datensätze zueinander müssen - wie in Figur 5a dargestellt - innerhalb eines Toleranzdreiecks mit der Seitenlänge "Max. Limit" liegen, wobei "Max. Limit" die in einer Norm vorgeschriebene Toleranzgrenze beschreibt. Figur 5b zeigt eine Darstellung für ein akzeptables, validiertes Simulatorsystem, das sich innerhalb des Toleranzdreiecks befindet. Alle drei Datensets - IBP (Gold-Standard), CNAP sowie 1st SIMU - haben untereinander Differenzen, die innerhalb der Toleranzgrenzen sind. In Figur 5c ist dies nicht der Fall, weil hier z.B. die Abweichung zwischen IBP und 1st SIMU zu groß ist.

Obwohl für die ersten Simulationen die genau gleichen CNAP-Komponenten (Sensoren, Lichtsystem, Regelsystem, Anpressvorrichtung, etc.) verwendet werden, wird der 1st SIMU Blutdruckverlauf nicht komplett äquivalent zum CNAP-Verlauf sein. Dies ist deswegen der Fall, weil die Komparatorfunktion des Simulationsmoduls 210 natürlich nur nachgebildet wurde. Je besser CNAP und 1st SIMU korrelieren, desto genauer wurde die S-kurvenförmige Komparatorfunktion nachgebildet.

Das Testsystem wird aber nicht gebaut, um dieselben CNAP-Komponenten nachträglich zu testen. In Figur 5d wird nun gezeigt, wie man neue CNAP-Systeme oder Teilsysteme testen kann und vor allem welche Toleranzgrenzen für Validierungen eingehalten werden müssen. Wenn ein neues Datenset mit neuen CNAP- Komponenten erzeugt wird, indem wiederum IBP als der Gold-Standard in den Simulator mit den neuen CNAP- Komponenten eingespielt wird, dann muss das neue Datensystem zu allen drei Datensets - IBP (Gold-Standard), CNAP sowie 1st SIMU -Differenzen innerhalb der Toleranzgrenze aufweisen. Anders gesagt, das neue SIMU-Datenset muss innerhalb eines Tetraeders liegen, der sich gemäß Figur 5d über dem aus Figur 5a bekannten "Toleranzdreieck" mit der Seitenlänge "Max. Limit" aufspannt.

## Patentansprüche

**1.** Verfahren zur Validierung eines kontinuierlich messenden, nicht-invasiven Blutdruckmesssystems (202), das mit einem plethysmographischen System ausgestattet ist, das geeignet ist - in einer Messphase - ein plethysmographisches Signal v(t) an einer Extremität (101) zu gewinnen,

- wobei das Signal v(t) aus dem plethysmographischen System, aufweisend zumindest eine Lichtquelle (203) und zumindest einen Lichtdetektor (204), einem Regelmechanismus (206) zugeführt wird, welcher über einen Stellwert u(t) den Anpressdruck pc(t) des Blutdruckmesssystems (202) an der Extremität (101) verändert,
- wobei basierend auf dem resultierenden Anpressdruck $p_c(t)$ der Verlauf des arteriellen Blutdrucks $p_A(t)$ kontinuierlich bestimmt wird,

**dadurch gekennzeichnet,**

- **dass** - in einer Validierungs- oder Testphase - ein Signal, abgeleitet von einem zuvor aufgezeichneten Blutdruckverlauf über eine Einkoppelschnittstelle (209) in das Blutdruckmesssystem (202) eingespeist wird, wobei die Lichtstärke der zumindest einen Lichtquelle (203) basierend auf dem zuvor aufgezeichneten Blutdruckverlauf moduliert wird, sowie
- **dass** der Einkoppelschnittstelle (209) ein Ausgangssignal aus einem Simulationsmodul (210) zugeführt wird, welchem ein auf einem Speichermedium (211) aufgezeichneter Blutdruckverlauf sowie der Anpressdruck pc(t) des Blutdruckmesssystems (202) als Eingangssignale zugeführt werden, wobei durch das Blutdruckmesssystem (202) ein simulierter, kontinuierlicher Blutdruckverlauf bestimmt wird.

**2.** Verfahren nach Anspruch 1, **dadurch gekennzeichnet, dass** der simulierte, kontinuierliche Blutdruckverlauf mit dem aus dem Speichermedium (211) in das Simulationsmodul (210) eingekoppelten Blutdruckverlauf verglichen wird und Unterschiede zwischen dem gespeicherten und dem simulierten Blutdruckverlauf als Kriterium zur Validierung des Blutdruckmesssystems (202) herangezogen werden.

**3.** Verfahren nach Anspruch 1 oder 2, **dadurch gekennzeichnet, dass** der zuvor aufgezeichnete Blutdruckverlauf für die Validierung des Blutdruckmesssystems (202) von einer intra-arterielle Blutdruckmessung (IBP) oder einer nicht-invasiven Blutdruckmessung (CNAP) einer vorrangegangenen klinischen Messung gewonnen wird.

**4.** Verfahren nach einem der Ansprüche 1 bis 3, **dadurch gekennzeichnet, dass**

- zeitgleich zu einer nicht-invasiven Blutdruckmessung (CNAP) an einem Lebewesen eine intra-arterielle Blutdruckmessung (IBP) am selben Lebewesen durchgeführt und aufgezeichnet wird;
- dass der aufgezeichnete intra-arterielle Blutdruckverlauf (IBP) und/oder der aufgezeichnete nicht-invasiven Blutdruckverlauf (CNAP) in das Blutdruckmesssystem (202) eingekoppelt werden und durch das Blutdruckmesssystem (202) simulierte Blutdruckverläufe (SIMU) erzeugt werden; und
- dass die simulierten Blutdruckverläufe (SIMU) als Kriterium zur Validierung des Blutdruckmesssystems (202) herangezogen werden.

**5.** Verfahren nach einem der Ansprüche 1 bis 4, **dadurch gekennzeichnet, dass** elektronische Elemente des Blutdruckmesssystems (202), wie beispielsweise das plethysmographische System, oder Mittel zur Veränderung des Anpressdruckes pc(t) Systems - in der Validierungs- oder Testphase - als digitales Softwaremodell nachgebildet werden.

**6.** Verfahren nach einem der Ansprüche 1 bis 5, **dadurch gekennzeichnet, dass** im Simulationsmodul (210) eine S-förmige oder glockenkurvenförmige Komparatorfunktion berechnet wird.

**7.** Verfahren nach Anspruch 6, **dadurch gekennzeichnet, dass** die S-förmige oder glockenkurvenförmige Komparatorfunktion vor Beginn der Datenaufzeichnung zur Blutdruckmessung in einer Open-Loop-Phase des Blutdruckmesssystems (202) bestimmt wird.

**8.** Verfahren nach Anspruch 7, **dadurch gekennzeichnet, dass** die S-förmige oder glockenkurvenförmige Komparatorfunktion während der Datenaufzeichnung der Blutdruckmessung überprüft und gegebenenfalls korrigiert wird.

**9.** Vorrichtung zur Validierung eines kontinuierlich messenden, nicht-invasiven Blutdruckmesssystems (202), das mit einem plethysmographischen System ausgestattet ist, das zumindest eine Lichtquelle (203) und zumindest einen Lichtdetektor (204) aufweist und geeignet ist - in einer Messphase - ein plethysmographisches Signal v(t) an einer Extremität (101) zu gewinnen,

- mit einem Regelmechanismus (206) dem das Signal v(t) aus dem plethysmographischen System zugeführt wird, welcher über einen Stellwert u(t) den Anpressdruck pc(t) an der Extremität (201) verändert, und
- mit einer Auswerteeinheit, welche basierend auf dem resultierenden Anpressdruck $p_c$(t) den Verlauf des arteriellen Blutdrucks $p_A$(t) kontinuierlich bestimmt.

**dadurch gekennzeichnet, dass**

- das Blutdruckmesssystem (202) eine Einkoppelschnittstelle (209) aufweist, über die - in einer Validierungs- oder Testphase - ein Signal, abgeleitet von einem zuvor aufgezeichneten Blutdruckverlauf in das Blutdruckmesssystem (202) einkoppelbar ist, wobei die Einkoppelschnittstelle (209) geeignet ist, die Lichtstärke der Lichtquelle (203) basierend auf dem Signal des aufgezeichneten Blutdruckverlaufs zu modulieren, sowie
- dass das Blutdruckmesssystem (202) ein Simulationsmodul (210) aufweist, das eingangsseitig einerseits mit einem Speichermedium (211) verbunden ist, das ein Signal des zuvor aufgezeichneten Blutdruckverlaufs $p_A$(t) bereit stellt und andererseits mit einer Druckerzeugungseinrichtung (208) des plethysmographischen Systems, die ein Signal des Anpressdrucks pc(t) bereit stellt, wobei das Simulationsmodul (210) geeignet ist, basierend auf den beiden Eingangssignalen eine Komparatorfunktion zu berechnen und an die Einkoppelschnittstelle (209) zu übermitteln.

**10.** Vorrichtung nach Anspruch 9, **dadurch gekennzeichnet, dass** die zumindest eine Lichtquelle (203) eine LED sowie der zumindest eine Lichtdetektor (204) eine Fotodiode aufweist.

**11.** Vorrichtung nach Anspruch 9 oder 10, **dadurch gekennzeichnet, dass** die Vorrichtung ein Fingerdummy (201) aufweist, das in der Validierungs- oder Testphase des Blutdruckmesssystems (202) - anstelle der Extremität (101) in der Messphase - in Kontakt mit dem plethysmographischen System bringbar ist.

**12.** Vorrichtung nach Anspruch 11, **dadurch gekennzeichnet, dass** die haptischen und/oder optischen Eigenschaften des Fingerdummies (201) der Extremität (101) eines Lebewesens entsprechen.

**Claims**

**1.** Method for validating a continuously measuring, non-invasive blood pressure measuring system (202) equipped with a plethysmographic system which is suitable for obtaining - in a measuring phase - a plethysmographic signal v(t) at an extremity (101),

- wherein the signal v(t) from the plethysmographic system, having at least one light source (203) and at least one light detector (204), is fed to a control mechanism (206) which changes the contact pressure pc(t) of the blood pressure measuring system (202) at the extremity (101) via a contol value u(t),

• wherein based on the resulting contact pressure pc(t) the arterial blood pressure curve $p_A$(t) is continuously determined,

**characterised in**

• **that** - in a validation or test phase - a signal derived from a previously recorded blood pressure curve is fed into the blood pressure measuring system (202) via a coupling interface (209), wherein the light intensity of at least one light source (203) is modulated based on the previously recorded blood pressure curve, and

• **that** an output signal from a simulation module (210) is fed to the coupling interface (209), to which a blood pressure curve recorded on a storage medium (211) and the contact pressure pc(t) of the blood pressure measuring system (202) are fed as input signals, wherein a simulated, continuous blood pressure curve is determined by the blood pressure measuring system (202).

2. Method according to claim 1, **characterised in that** the simulated, continuous blood pressure curve is compared with the blood pressure curve coupled from the storage medium (211) into the simulation module (210) and differences between the stored and the simulated blood pressure curve are used as a criterion for validating the blood pressure measuring system (202).

3. Method according to claim 1 or 2, **characterised in that** the previously recorded blood pressure curve for the validation of the blood pressure measuring system (202) is obtained from an intra-arterial blood pressure measurement (IBP) or a non-invasive blood pressure measurement (CNAP) of a preceding clinical measurement.

4. Method according to one of claims 1 to 3, **characterised in that**

• at the same time as a non-invasive blood pressure measurement (CNAP) in a living being, an intra-arterial blood pressure measurement (IBP) is performed and recorded in the same living being;

• that the recorded intra-arterial blood pressure curve (IBP) and/or the recorded non-invasive blood pressure curve (CNAP) are coupled into the blood pressure measuring system (202) and blood pressure curves (SIMU) simulated by the blood pressure measuring system (202) are generated; and

• that the simulated blood pressure curves (SIMU) are used as a criterion for validating the blood pressure measurement system (202).

5. Method according to one of claims 1 to 4, **characterised in that** electronic elements of the blood pressure measuring system (202), such as the plethysmographic system, or means for changing the contact pressure pc(t) system - in the validation or test phase - are simulated as a digital software model.

6. Method according to one of claims 1 to 5, **characterised in that** an S-shaped or bell-curve comparator function is calculated in the simulation module (210).

7. Method according to claim 6, **characterised in that** the S-shaped or bell-shaped comparator function is determined in an open-loop phase of the blood pressure measuring system (202) before the start of data recording for blood pressure measurement.

8. Method according to claim 7, **characterised in that** the S-shaped or bell-curve comparator function is checked and, if necessary, corrected during the data recording of the blood pressure measurement.

9. Apparatus for validating a continuously measuring, non-invasive blood pressure measuring system (202) equipped with a plethysmographic system having at least one light source (203) and at least one light detector (204) and is capable of obtaining - in a measuring phase - a plethysmographic signal v(t) at an extremity (101),

• having a control mechanism (206) to which the signal v(t) from the plethysmographic system (203, 204) is fed, which changes the contact pressure pc(t) at the extremity (201) via a control value u(t), and

• having an evaluation unit which continuously determines the arterial blood pressure curve $p_A$(t) based on the resulting contact pressure $p_c$(t).

**characterised in that**

• the blood pressure measuring system (202) has a coupling interface (209) via which - in a validation or test phase - a signal derived from a previously recorded blood pressure curve can be input into the blood pressure measuring system (202), wherein the coupling interface (209) is adapted to modulate the light intensity of the light source (203) based on the signal of the recorded blood pressure curve, and

• the blood pressure measuring system (202) comprises a simulation module (210) which is connected on the input side on the one hand to a storage medium (211) which provides a signal of the previously recorded blood pressure curve $p_A(t)$ and on the other hand to a pressure generating device (208) of the plethysmographic system, which provides a signal of the contact pressure pc(t), wherein the simulation module (210) is suitable for calculating a comparator function based on the two input signals and transmitting it to the coupling interface (209).

10. Apparatus according to claim 9, **characterised in that** the at least one light source (203) comprises an LED and the at least one light detector (204) comprises a photodiode.

11. Apparatus according to claim 9 or 10, **characterised in that** the apparatus comprises a finger dummy (201) which can be brought into contact with the plethysmographic system in the validation or test phase of the blood pressure measuring system (202) instead of the extremity (101) in the measuring phase.

12. Apparatus according to claim 11, **characterised in that** the haptic and/or optical properties of the finger dummy (201) correspond to the extremity (101) of a living being.

**Revendications**

1. Procédé de validation d'un système de mesure de la pression artérielle (202) non invasif, mesurant en continu, et équipé d'un système pléthys-mographique, approprié pour -dans une phase de mesure- obtenir un signal pléthys-mographique v(t) à une extrémité de membre (101),

    * le signal v(t) du système pléthysmographique comprenant au moins une source lumineuse (203) et au moins un photodétecteur (204) appliqué à un mécanisme de régulation (206) qui modifie la pression de contact pc(t) du système de mesure de la pression artérielle (202) à l'extrémité de membre (101) par une valeur de réglage u(t), et

    * en se fondant sur la pression de contact pc(t) résultante, on détermine en continu la courbe de la pression artérielle $p_A(t)$,

    procédé **caractérisé en ce que**

    * on injecte dans une phase de validation ou de test- un signal d'une courbe de pression artérielle enregistrée préalablement, par une interface de couplage (209) dans le système de mesure de pression artérielle (202), l'intensité lumineuse de cette source de lumière (203) étant modulée en fonction de la courbe de pression artérielle enregistrée préalablement, et

    * on applique à l'interface de couplage (209) un signal de sortie d'un module de simulation (210) qui reçoit comme signaux d'entrée, une courbe de pression artérielle enregistrée sur un support de mémoire (211) ainsi que la pression de contact pc(t) du système de mesure de pression artérielle (202), le système de mesure de pression artérielle (202) déterminant une courbe de pression artérielle, simulée, continue.

2. Procédé selon la revendication 1,
**caractérisé en ce que**
on compare la courbe de pression artérielle simulée, continue à la courbe de pression enregistrée provenant du support de mémoire (211) dans le module de simulation (210) et on utilise les différences entre la courbe de pression artérielle enregistrée et celle simulée, comme critère de validation du système de mesure de pression artérielle (202).

3. Procédé selon la revendication 1 ou 2,
**caractérisé en ce que**
on obtient la courbe de pression artérielle enregistrée préalablement pour la validation du système de mesure de pression artérielle (202) à partir d'une mesure de pression intra-artérielle (IBP) ou d'une mesure de pression artérielle, non-invasive (CNAP) d'une mesure clinique antérieure.

4. Procédé selon l'une des revendications 1 à 3,
**caractérisé en ce que**

* simultanément à une mesure de pression artérielle non invasive (CNAP) sur un être humain on effectue une mesure de pression intra-artérielle (IBP) sur le même être vivant et on l'enregistre ;
* on injecte la courbe de pression intra-artérielle enregistrée (IBP) et/ou la courbe de pression artérielle non invasive (CNAP) enregistrée, dans le système de mesure de pression artérielle (202) et on génère des courbes de pression artérielle simulées (SIMU) par le système de mesure de pression artérielle (202) ; et
* on utilise les courbes de pression artérielle simulées (SIMU) comme critère de validation du système de mesure de pression artérielle (202).

**5.** Procédé selon l'une des revendications 1 à 4,
**caractérisé en ce que**
on copie les éléments électroniques du système de mesure de pression artérielle (202) comme, par exemple, le système pléthysmographique ou des moyens pour modifier le système de pression de contact pc(t) - dans la phase de validation ou la phase de test - comme modèle de programme numérique.

**6.** Procédé selon l'une des revendications 1 à 5,
**caractérisé en ce que**
dans le module de simulation (210), on calcule une fonction de comparaison en forme de S ou en forme de cloche.

**7.** Procédé selon la revendication 6,
**caractérisé en ce que**
avant le début de l'enregistrement des données pour la mesure de la pression artérielle, on détermine la fonction de comparaison en forme de S ou en forme de cloche dans une phase en boucle ouverte du système de mesure de pression artérielle (202).

**8.** Procédé selon la revendication 7,
**caractérisé en ce que**
on vérifie la fonction de comparaison en forme de S ou de cloche pendant l'enregistrement des données de la mesure de pression artérielle et le cas échéant on la corrige.

**9.** Dispositif de validation d'un système de mesure de pression artérielle (202) non invasif, mesurant en continu, qui est équipé d'un système pléthysmographique, comprenant au moins une source de lumière (203) et au moins un photodétecteur (204) approprié pour :

- dans une phase de mesure - obtenir un signal pléthysmographique v(t) à une extrémité de membre (101) et avec,

* un mécanisme de régulation (206) qui reçoit le signal v(t) du système pléthysmographique et qui modifie la pression de contact pc(t) à l'extrémité (201) par une valeur de réglage u(t), et
* une unité d'exploitation qui détermine en continu la courbe de la pression artérielle $p_A(t)$ résultant de la pression de contact $p_c(t)$,
dispositif **caractérisé en ce que**
* le système de mesure de pression artérielle (202) comporte une interface de couplage (209) par laquelle dans une phase de validation ou une phase de test- on injecte un signal déduit d'une courbe de pression artérielle enregistrée au préalable dans le système de mesure de pression artérielle (202), l'interface de couplage (209) étant appropriée pour moduler l'intensité lumineuse de la source de lumière (203) en se fondant sur le signal de la courbe de pression artérielle enregistrée, et
* le système de mesure de pression artérielle (202) comporte un module de simulation (210) relié côté entrée d'une part à un support de mémoire (211) qui fournit un signal d'une courbe de pression artérielle $p_A(t)$ enregistrée au préalable et d'autre part, avec une installation générant une pression (208) du système pléthysmographique qui fournit un signal de pression de contact pc(t), le module de simulation (210) étant approprié pour calculer une fonction de comparaison fondée sur l'un des deux signaux d'entrée et transmettre le signal à une interface de couplage (209).

**10.** Dispositif selon la revendication 9,
**caractérisé en ce que**
au moins la source lumineuse (203) comporte une LED et au moins le photodétecteur (204) comporte une photodiode.

**11.** Dispositif selon la revendication 9 ou 10,
**caractérisé en ce que**

le dispositif comprend un doigt factice (201) qui est mis en phase de validation ou de test du système de mesure de pression (202) contact avec le système pléthysmographique -à la place de l'extrémité (101) en phase de mesure-.

12. Dispositif selon la revendication 11,
   **caractérisé en ce que**
   les caractéristiques haptiques et/ou optiques du doigt factice (201) correspondent à celles de l'extrémité (101) d'un être vivant.

Fig. 1

$p_A(t)$

210

211

202

209

203

$p_C(t)$  208

201

204  205

206

u(t)

v(t)

Fig. 2

EP 3 796 834 B1

16

Fig. 3a

Fig. 3b

Fig. 4a

EP 3 796 834 B1

Fig. 4b

EP 3 796 834 B1

Fig. 5a

Fig. 5b

Fig. 5c

Fig. 5d

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4510940 A
- US 8114025 B2
- US 8814800 B2
- US 10098554 B2
- WO 2016110781 A
- WO 2016110781 A1
- WO 2011051819 A1
- US 8343062 B2

**In der Beschreibung aufgeführte Nicht-Patentliteratur**

- **JAN PENAZ.** *Digest of the 10th International Conference on Medical and Biological Engineering 1973 Dresden,* 1973